(19) **Europäisches Patentamt European Patent Office Office européen des brevets**

(11) **EP 3 483 603 B1**

(12) **EUROPEAN PATENT SPECIFICATION**

(45) Date of publication and mention of the grant of the patent:
**02.08.2023 Bulletin 2023/31**

(21) Application number: **17824053.7**

(22) Date of filing: **26.06.2017**

(51) International Patent Classification (IPC):
**G01N 30/88** (2006.01)   **G01N 30/06** (2006.01)
**G01N 33/49** (2006.01)   **G01N 33/72** (2006.01)

(52) Cooperative Patent Classification (CPC):
**G01N 33/721;** G01N 2030/8822

(86) International application number:
**PCT/JP2017/023394**

(87) International publication number:
**WO 2018/008447 (11.01.2018 Gazette 2018/02)**

(54) **HEMOGLOBIN LIQUID PREPARATION AND LIQUID CHROMATOGRAPHY METHOD FOR MEASURING HEMOGLOBIN COMPONENT**

FLÜSSIGES HÄMOGLOBINPRÄPARAT UND FLÜSSIGCHROMATOGRAFIEVERFAHREN ZUR MESSUNG VON HÄMOGLOBINBESTANDTEILEN

PRÉPARATION LIQUIDE D'HÉMOGLOBINE ET PROCÉDÉ DE CHROMATOGRAPHIE LIQUIDE POUR MESURER LE COMPOSANT D'HÉMOGLOBINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2016 JP 2016136270**

(43) Date of publication of application:
**15.05.2019 Bulletin 2019/20**

(73) Proprietor: **Tosoh Corporation Shunan-shi, Yamaguchi 746-8501 (JP)**

(72) Inventors:
• **SAKO Miyuki**
  **Shunan-shi**
  **Yamaguchi 746-8501 (JP)**
• **NAKAZAWA Yuji**
  **Shunan-shi**
  **Yamaguchi 746-8501 (JP)**

(74) Representative: **TBK**
  **Bavariaring 4-6**
  **80336 München (DE)**

(56) References cited:
EP-A1- 2 360 471     JP-A- H02 259 467
JP-A- H06 308 120     JP-A- H06 331 629
JP-A- 2009 133 654     US-A- 6 150 507

• KERWIN B A ET AL: "EFFECTS OF TWEEN 80 AND SUCROSE ON ACUTE SHORT-TERM STABILITY AND LONG-TERM STORAGE AT -20°C OF A RECOMBINANT HEMOGLOBIN", JOURNAL OF PHARMACEUTICAL SCIENCES, AMERICAN PHARMACEUTICAL ASSOCIATION, US, vol. 87, no. 9, 1 September 1998 (1998-09-01), pages 1062-1068, XP000773113, ISSN: 0022-3549, DOI: 10.1021/JS980140V
• C G DUCK-CHONG: "Differential effect of detergents on the alkaline denaturation of haemoglobin in maternal and fetal blood, with particular reference to Triton X-100.", JOURNAL OF CLINICAL PATHOLOGY, vol. 36, no. 8, 1 August 1983 (1983-08-01) , pages 910-914, XP055665497, GB ISSN: 0021-9746, DOI: 10.1136/jcp.36.8.910

**Description**

Technical Field

The present invention relates to the use of a preparation solution for analyzing hemoglobin in a blood sample and a liquid chromatography method for measuring hemoglobins using the preparation solution.

Background Art

The concentration of stable glycosylated hemoglobin (HbA1c) formed by nonenzymatic bonding (glycation) with glucose in the blood changes in response to the change in blood glucose level in the past two or three months without being affected by a temporal increase in blood glucose level due to meals or the like. Therefore, stable glycosylated hemoglobin is widely used as a marker for diagnosis of diabetes and follow-up of diabetic patients.

A cation-exchange liquid chromatography method (hereafter abbreviated as "HPLC method") is widely used for the measurement of HbA1c because the method is excellent in terms of accuracy and reproducibility. In general, HbA1c measurement is carried out as follows. The performance of the instrument calibrated with calibrators is checked by controls and then measurements with blood samples are carried out. The calibration of an instrument with a calibrator is performed when necessary, for example, during column replacement or during maintenance (PTL 1).

In the liquid chromatography method, the calibration of an instrument and the quality control are ideally performed under the same conditions as those of actual measurement in terms of reliability of measurement results. In other words, it is known that all hemoglobin samples such as calibrators, controls, and blood samples are desirably prepared by using the same preparation solution.

Currently used preparation solutions can be used for blood samples, but sometimes cannot be used for lyophilized samples because, for example, a new component (peak) appears between HbA1c and HbAO, which interfere the accuracy of the measurement, and the HbA1c concentration changes because of poor stability after preparing.

Citation List

Patent Literature

PTL 1: Japanese Patent No. 4061365

Summary of Invention

JP 6-331629 A describes a technology for measuring hemoglobin in blood samples by using liquid chromatography.

JP 2009-133654 A mentions the measurement of hemoglobins by using liquid chromatography.

KERWIN B A et al., "EFFECTS OF TWEEN 80 AND SUCROSE ON ACUTE SHORT-TERM STABILITY AND LONG-TERM STORAGE AT - 20°C OF A RECOMBINANT HEMOGLOBIN", JOURNAL OF PHARMACEUTICAL SCIENCES, AMERICAN PHARMACEUTICAL ASSOCIATION, US, (19980901), vol. 87, no. 9, pages 1062 - 1068 describes effects of Tween 80 and sucrose on stability and storage of a recombinant hemoglobin.

EP 2 360 471 A1 describes a method for pre-treating a sample containing a glycosylated hemoglobin, wherein a guanidine or a salt thereof as well as a non-ionic surfactant and/or a nitrile is used.

C G DUCK-CHONG, "Differential effect of detergents on the alkaline denaturation of haemoglobin in maternal and fetal blood, with particular reference to Triton X-100.", JOURNAL OF CLINICAL PATHOLOGY, GB, (19830801), vol. 36, no. 8, pages 910 - 914 investigates the alkaline denaturation of hemoglobin in blood by using Triton X-100 as well as Brij-35, Tween 80 and Nonidet P40.

Technical Problem

It is an object of the present invention to provide the use of a hemoglobin preparation solution with which all samples containing hemoglobin, such as a calibrator, a control, and a blood sample, can be prepared regardless of the state of the samples containing hemoglobin, and a measurement method that uses the hemoglobin preparation solution.

Solution to Problem

As a result of thorough studies to achieve the above object, the present inventors have found the following. The reason why the HbA1c value is unstable after hemoglobin-containing samples are prepared using preparation solutions are that ether-type nonionic surfactants contained in the preparation solutions affect hemoglobins contained in HbA0 fraction and ester ether-type nonionic surfactants do not affect the hemoglobins. Thus, the present invention has been completed.

The details of the present invention are defined in the claims.

The hemoglobin-containing samples according to the present invention are any hemoglobin-containing samples including but not limited to lyophilized hemoglobins, frozen hemoglobin liquids, or blood samples. Furthermore, The hemoglobin-containing samples according to the present invention contain the processed samples such as lyophilized hemoglobin or frozen hemoglobin liquid that are adjusted the components by removing blood plasma components or adding saccharides or antiseptic to ensure their stability.

The preparation solution for measuring hemoglobins according to the present invention is a preparation solution that contains at least one ester ether-type nonionic surfactant, but does not contain any ether-type nonionic surfactants.

The preparation process according to the present invention is to adjust the concentration of the hemoglobin-containing sample to a desired concentration. Specifically, the concentration of the hemoglobin-containing sample, which is a calibrator, a control, or a blood sample, is adjusted to the desire concentration to inject the sample into column by using the preparation solution

The ester ether-type nonionic surfactant according to the present invention is a compound obtained by adding ethylene oxide to a fatty acid ester. Examples of the compounds include polyoxyethylene sorbitan monolaurate, polyoxyethylene monostearate, polyoxyethylene sorbitan tristearate, and polyoxyethylene sorbitan monooleate each obtained by adding ethylene oxide to a fatty acid ester of polyhydric alcohol. The average number (n) of moles of ethylene oxide added is not particularly limited, but polyoxyethylene (20) sorbitan monolaurate, polyoxyethylene (20) monostearate, polyoxyethylene (20) sorbitan tristearate, and polyoxyethylene sorbitan monooleate (20) that satisfy n = 20 are particularly preferred.

The ether-type nonionic surfactant according to the present invention is, for example, a polyoxyethylene alkylaryl ether obtained by performing addition polymerization with ethylene oxide on an alkylphenol, a polyoxyethylene alkyl ether obtained by performing addition polymerization with ethylene oxide on a higher alcohol, or a polyoxyethylene polyoxypropylene glycol obtained by performing addition polymerization with ethylene oxide on polypropylene glycol. Non-limiting examples of the ether-type nonionic surfactant include polyoxyethylene (8) octylphenyl ether, polyoxyethylene (10) octylphenyl ether, and polyethylene (23) lauryl ether.

The concentration of the ester ether-type nonionic surfactant is 0.05 to 0.4 wt%.

The concentration herein is a concentration after the hemoglobin-containing sample is prepared using the preparation solution. If the concentration is lower than 0.01 wt%, hydrophobic components in hemoglobin samples remain on the instruments, columns, or the like. The pollution of them cannot be prevented. If the ester ether-type nonionic surfactants have absorptivity to 415 nm and the concentration of them exceed 0.4 wt%, a peak derived from the surfactants is sometimes detected in the front of HbA1a peak. The large of the peaks derived from surfactants are depending on the volume of the hemoglobin sample introduced into a column. But the detection at 415 nm is typical for detection of hemoglobin and the peak derived from surfactants interferes the accuracy of the measurement.

The preparation solution for measuring hemoglobins according to the present invention can contain at least one preservative to improve the preservation of the preparation solution. Compounds having an antimicrobial effect and an antiseptic effect can be used for the preservations. The preservative is preferably a chelate preservative such as ethylenediaminetetraacetic acid (hereafter, EDTA) or sodium azide. Alcohol compounds are not desirable because the concentration readily changes through volatilization. The chelate preservatives are including but not limited to EDTA, glycol ether diaminetetraacetic acid (hereafter, GEDTA), sodium salts of EDTA, potassium salts of EDTA, ethylenediamine-N,N'-disuccinic acid, and sodium salts of ethylenediamine-N,N'-disuccinic acid. The concentration of the preservative is 0.05 to 6 wt% and preferably 0.1 to 3 wt%. The concentration herein is a concentration after the hemoglobin-containing sample is prepared using the preparation solution.

The method for preparing a hemoglobin-containing sample using the preparation solution for measuring hemoglobins according to the present invention is, for example, a method in which lyophilized hemoglobin is dissolved and diluted by adding the preparation solution, a method in which a hemoglobin solution primarily diluted with a small amount of purified water is further secondarily diluted with the preparation solution, or a method in which a small amount of thawed liquid of frozen hemoglobin or blood sample is added to the preparation solution for dilution. The method is not particularly limited as long as the hemoglobin-containing sample is prepared so that the sample can be analyzed by a liquid chromatography method.

When the preparation solution for measuring hemoglobins is used for preparing a hemoglobin sample to be measured by a cation-exchange liquid chromatography method, the cation concentration of the hemoglobin preparation solution affects the measurement. The concentration of cations excluding a hydrogen ion in the hemoglobin preparation solution is in the range of 5 to 40 mM and the pH is preferably 5.0 to 9.5.

If the pH is less than 5.0 or more than 9.5, the peak shape of each hemoglobin in the hemoglobin sample changes and HbA1c % cannot be stably measured. If the concentration of cation excluding hydrogen ion, which is derived from the preservatives and pH adjuster, is less than 5mM, the tubes of the instrument are polluted by the substances of the sample as a result of long-terms use. This pollution interfered the reliability of the measurement. If the concentration is more than 40 mM, the separation of hemoglobins such as HbA1a, HbA1b, and HbF that have a weaker interaction with a filler than HbA1c become poor, and thus HbA1c % cannot be stably measured.

A buffer such as phosphoric acid or MES can be added to stabilize the pH as long as the total concentration of cations other than a hydrogen ion is within 5 to 40 mM. It gives advantageous effects of this invention.

When the preparation solution for measuring hemoglobins according to the present invention is used, hemoglobin can be stably measured without causing variations in the retention time and area of hemoglobin components. Furthermore, all hemoglobin samples that can be measured by a liquid chromatography method can be prepared by a using same preparation solution. Therefore, calibration of an instrument, quality control, and blood sample measurement can be precisely performed.

Brief Description of Drawings

[Fig. 1] Fig. 1 illustrates chromatograms of lyophilized hemoglobin-A in Example 1.
[Fig. 2] Fig. 2 illustrates chromatograms of lyophilized hemoglobin-A in Comparative Example 1.
[Fig. 3] Fig. 3 illustrates chromatograms of lyophilized hemoglobin-B in Example 3, Comparative Example 3, and Comparative Example 4.

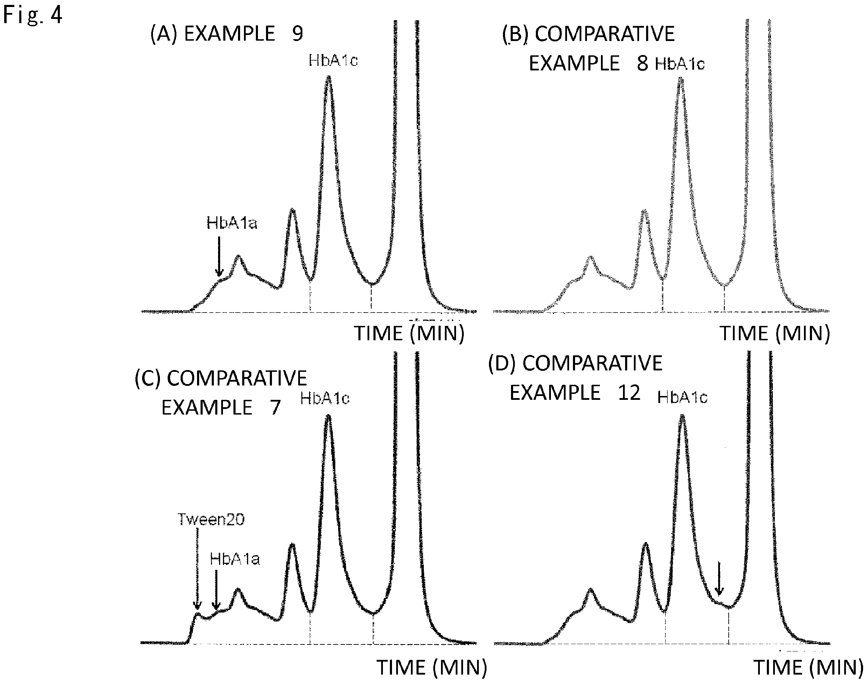

[Fig. 4] Fig. 4 illustrates chromatograms of lyophilized hemoglobin-M in Example 9, Comparative Example 7, Comparative Example 8, and Comparative Example 12.

Description of Embodiments

Hereafter, the present invention will be described in detail based on Examples and Comparative Examples. The present invention is not limited to Examples below.

(Example 1)

A hemoglobin sample was prepared by the following method, and the change in chromatograms after dilution was observed. Table 1 shows the results.

(1) Production of hemoglobin preparation solution

In purified water, 0.1 wt% of polyoxyethylene (20) sorbitan monolaurate (hereafter, Tween 20) serving as a surfactant, 0.1 wt% of sodium azide serving as a preservative, 0.05 wt% of tetrasodium EDTA tetrahydrate serving as a preservative, and 0.05 wt% of dipotassium EDTA dihydrate serving as a preservative were dissolved to produce a hemoglobin preparation solution having a pH of 7.5.

(2) Preparation of hemoglobin sample

[I] To lyophilized hemoglobin-A, 500 $\mu$L of purified water was added and the resulting solution was left to stand at room temperature for 30 minutes to obtain a 40 g/L high-concentration hemoglobin solution. Twenty microliters of the high-concentration hemoglobin solution was diluted with 1000 $\mu$L of the hemoglobin preparation solution to obtain an about 0.8 g/L hemoglobin sample.

(3) Measurement

The hemoglobin sample was injected into a liquid chromatograph to which a cation exchange column was connected immediately after the dilution with the hemoglobin preparation solution, that is, within five minutes. Hemoglobin components were separated using succinate buffers having pH of 5.4 and detected at 415 nm. After the hemoglobin sample was left to stand at 25°C for 30 minutes, the measurement was performed again.

(Example 2)

The same process as in Example 1 was performed, except that polyoxyethylene (20) sorbitan monooleate (hereafter, Tween 80) was used as a surfactant instead of Tween 20 in Example 1.

(Comparative Example 1)

The same process as in Example 1 was performed, except that polyoxyethylene (10) octylphenyl ether (hereafter, Triton X-100) was used as a surfactant instead of Tween 20 in Example 1.

(Comparative Example 2)

The same process as in Example 1 was performed, except that polyoxyethylene (8) octylphenyl ether (hereafter, Triton X-114) was used as a surfactant instead of Tween 20 in Example 1.

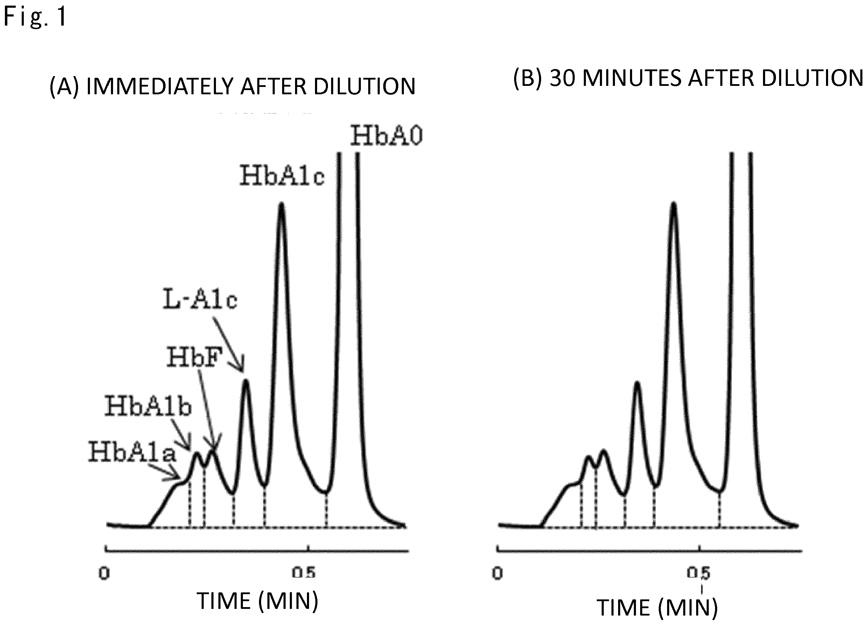

Fig. 1 illustrates chromatograms obtained in Example 1.

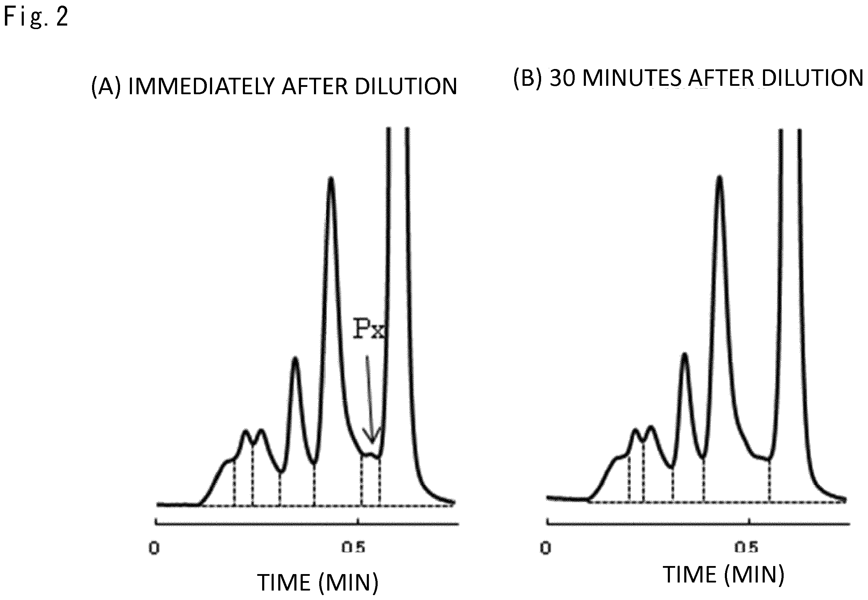

Fig. 2 illustrates chromatograms obtained in Comparative Example 1.

[Table 1]

|  | Example | | Comparative Example | |
|---|---|---|---|---|
|  | 1 | 2 | 1 | 2 |
| Surfactant | Tween 20 | Tween 80 | Triton X-100 | Triton X-114 |
| Immediately after dilution | No Px | No Px | Px appears | Px appears |
| 30 minutes after dilution | No Px | No Px | No Px | No Px |

In Comparative Example 1 and Comparative Example 2 in which an ether-type nonionic surfactant was contained, a peak Px was detected between HbA1c and HbA0 in the measurement immediately after the dilution. The peak Px was not detected 30 minutes after the dilution. This is because the elution time of a certain component of HbA0 was changed by the action of the ether-type nonionic surfactant. As shown in Table 1, Px was not detected in Example 1 and Example 2 in which an ester ether-type nonionic surfactant was contained, and the change in chromatograms over time was not observed.

(Example 3)

A hemoglobin sample was prepared by the following method, and HbA1c % was measured by a liquid chromatography method. Table 2 shows the results.

(1) Production of hemoglobin preparation solution

In purified water, 0.1 wt% of Tween 20 serving as a surfactant, 0.05 wt% of sodium azide serving as a preservative, 0.05 wt% of tetrasodium EDTA tetrahydrate serving as a preservative, and 0.05 wt% of dipotassium EDTA dihydrate serving as a preservative were dissolved to produce a hemoglobin preparation solution having a pH of 7.5.

(2) Preparation of hemoglobin sample

[I] Lyophilized hemoglobin-B

To lyophilized hemoglobin-B, 1000 μL of the hemoglobin preparation solution was added to dissolve the lyophilized hemoglobin-B. Thus, an about 0.8 g/L hemoglobin sample was obtained.

[II] Lyophilized hemoglobin-C

To lyophilized hemoglobin-C, 500 μL of purified water was added and the resulting solution was left to stand at room temperature for 30 minutes to obtain a 40 g/L high-concentration hemoglobin solution. Twenty microliters of the high-concentration hemoglobin solution was diluted with 1000 μL of the hemoglobin preparation solution to obtain an about 0.8 g/L hemoglobin sample.

[III] Frozen blood sample-I

A blood sample-I stored at -80°C for two months or more was left to stand at room temperature for 20 minutes for thawing. This blood sample was diluted with the hemoglobin preparation solution to obtain an about 1 g/L hemoglobin sample.

[IV] Frozen hemoglobin-J

A frozen hemoglobin-J stored at -80°C was left to stand at room temperature for 10 minutes for thawing. Six microliters of the thawed liquid was diluted with 1000 μL of the hemoglobin preparation solution to obtain an about 0.8 g/L hemoglobin sample.

(3) Measurement of HbA1c %

The hemoglobin sample was injected into a liquid chromatograph to which a cation exchange column was connected immediately after the dissolution or dilution with the hemoglobin preparation solution, that is, within five minutes. Hemoglobin components were separated by a pH gradient method that uses a succinate buffer having pH of 5.4 and a succinate-phosphate buffer having pH of 6.3 and detected at 415 nm. HbA1c % was calculated from calculation formula A below.

```
Calculation formula A: HbA1c % = Area of HbA1c/(HbA1a +

HbA1b + L-A1c + HbA1c + HbA0) × 100
```

(Comparative Example 3)

A hemoglobin sample was prepared by the following method, and HbA1c % was measured by a liquid chromatography method. Table 2 shows the results.

(1) Production of hemoglobin preparation solution

In purified water, 0.05 wt% of sodium azide, 0.05 wt% of tetrasodium EDTA tetrahydrate, and 0.05 wt% of dipotassium EDTA dihydrate were dissolved to produce a hemoglobin preparation solution having a pH of 7.5 and not containing a surfactant.
The preparation of a hemoglobin sample (2) and the measurement of HbA1c % (3) were performed in the same manner as in Example 3.

(Comparative Example 4)

The same process as in Example 3 was performed, except that polyethylene (23) lauryl ether (hereafter, Brij 35) was used as a surfactant.

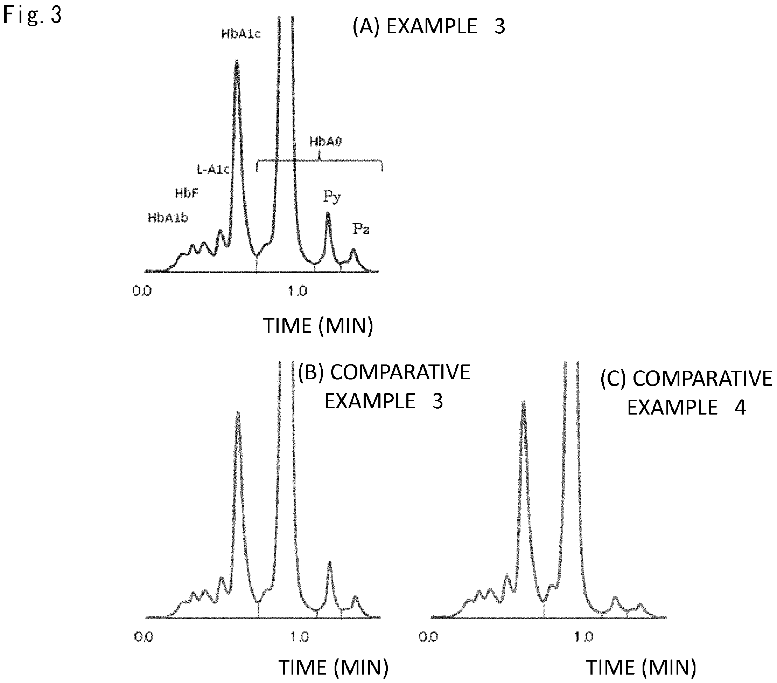

Fig. 3 illustrates chromatograms of the lyophilized hemoglobin-B. As is clear from the chromatograms in Fig. 3, when the separation of lyophilized hemoglobin is performed by a pH gradient method, Py and Pz are observed in the HbA0 fraction. The chromatogram and HbA1c % in Comparative Example 4 are totally different from those in Example 3 and Comparative Example 3. For example, HbA1c % is higher by 0.3% and the peak area of Py is obviously small. This shows that unlike Tween 20, Brij 35 affects the separation of hemoglobin components.
On the other hand, the frozen blood sample-I and the frozen hemoglobin-J were not affected by Brij 35.

[Table 2]

| | Example | Comparative Example | |
|---|---|---|---|
| | 3 | 3 | 4 |
| Surfactant | Tween 20 | - | Brij 35 |
| 1. Lyophilized hemoglobin-B | 9.1 | 9.1 | 9.4 |
| 2. Lyophilized hemoglobin-C | 7.9 | 7.9 | 8.2 |
| 3. Frozen blood sample-I | 8.6 | 8.6 | 8.7 |
| 4. Frozen hemoglobin-J | 8.8 | 8.7 | 8.9 |

(Example 4)

A hemoglobin sample was prepared by the following method, and the change in HbA1c % over time was investigated. Table 3 shows the results.

(1) Production of hemoglobin preparation solution

The same hemoglobin preparation solution as in Example 1 was produced.

(2) Preparation of hemoglobin sample

[I] Blood sample-D

A fresh blood sample that was collected within 24 hours and was not frozen even once was diluted with the hemoglobin preparation solution to obtain an about 1 g/L hemoglobin sample.

[II] Frozen blood sample-E

A blood sample stored at -80°C for two months or more was left to stand at room temperature for 20 minutes for thawing. The blood sample was diluted with the hemoglobin preparation solution to obtain an about 1 g/L hemoglobin sample.

(3) Measurement of HbA1c %

The hemoglobin sample was injected into a liquid chromatograph to which a cation exchange column was connected immediately after the dilution with the hemoglobin preparation solution, that is, within five minutes. Hemoglobin components were separated using succinate buffers having pH of 5.4 and detected at 415 nm. After the hemoglobin sample was left to stand at 25°C for 60 minutes from the dilution, the measurement was performed again.
HbA1c % was calculated from calculation formula A below.

$$\text{Calculation formula A: HbA1c \% = Area of HbA1c}/(\text{HbA1a} + \text{HbA1b} + \text{L-A1c} + \text{HbA1c} + \text{HbA0}) \times 100$$

(Example 5)

A hemoglobin sample was prepared by the following method, and the change in HbA1c % over time was investigated. Table 3 shows the results.

(1) Hemoglobin preparation solution

In purified water, 0.1 wt% of Tween 20 serving as a surfactant and 0.4 wt% of dipotassium EDTA dihydrate serving as a preservative were dissolved to produce a hemoglobin preparation solution. The pH of the hemoglobin preparation solution was adjusted to 6.0 by using sodium hydroxide.

The preparation of a hemoglobin sample (2) and the measurement of HbA1c % (3) were performed in the same manner as in Example 4.

(Example 6)

A hemoglobin sample was prepared by the following method, and the change in HbA1c % over time was investigated. Table 3 shows the results.

(1) Hemoglobin preparation solution

In purified water, 0.1 wt% of Tween 20 serving as a surfactant, 0.05 wt% of sodium azide serving as a preservative, 0.12 wt% of GEDTA serving as a preservative, and 0.04 wt% of sodium hydroxide for dissolving GEDTA were dissolved to produce a hemoglobin preparation solution having a pH of 9.3.

The preparation of a hemoglobin sample (2) and the measurement of HbA1c % (3) were performed in the same manner as in Example 4.

(Comparative Example 5)

A hemoglobin sample was prepared by the following method, and the change in HbA1c % over time was investigated. Table 3 shows the results.

(1) Production of hemoglobin preparation solution

The same hemoglobin preparation solution as in Comparative Example 3 was produced.

The preparation of a hemoglobin sample (2) and the measurement of HbA1c % (3) were performed in the same manner as in Example 4.

(Comparative Example 6)

A hemoglobin sample was prepared by the following method, and the change in HbA1c % over time was investigated. Table 3 shows the results.

(1) Production of hemoglobin preparation solution

The same hemoglobin preparation solution as in Comparative Example 1 was produced.

(2) Preparation of hemoglobin sample

The same process as in Example 4 was performed.

The measurement of HbA1c % (3) was performed in the same manner as in Example 4, except for the calculation formula of HbA1c %. When Px was not detected, HbA1c % was calculated from the calculation formula A as in Example 4. When Px was detected, HbA1c % was calculated from calculation formula B below.

```
Calculation formula B: HbA1c % = Area of HbA1c/(HbA1a +

HbA1b + L-A1c + HbA1c + Px + HbA0) × 100
```

As is clear from Example 4 to Example 6, in the blood sample diluted with the hemoglobin preparation solution containing Tween 20 as a surfactant, the chromatogram did not change for 60 minutes from the dilution without depending on the state of the blood sample, and thus HbA1c % was stably measurable.

Since the difference of HbA1c % are within -0.1% to 0.1% form Comparative Example 5 in which a surfactant is not contained, it is found that Tween 20 does not affect hemoglobin components.

In Comparative Example 6, the chromatogram and HbA1c % of the frozen blood sample-E changed over time unlike the fresh blood sample-D. In the frozen blood sample-E, Px was detected immediately after the dilution and HbA1c % calculated from the calculation formula B was lower than that in Comparative Example 5 by about -0.5%. Px was coeluted with the HbA1c fraction 60 minutes after the dilution, and HbA1c % calculated from the calculation formula A was 4.7%, which was higher than that in Comparative Example 5 by 0.3%. This shows that an HbA0 component deteriorated by freezing and thawing blood is easily affected by Triton X-100.

[Table 3]

| | | Example | | | Comparative Example | |
|---|---|---|---|---|---|---|
| | | 4 | 5 | 6 | 5 | 6 |
| 1. Blood sample-D | Immediately after dilution | 4.1 | 4.1 | 4.2 | 4.1 | 4.2 |
| | 60 minutes after dilution | 4.1 | 4.1 | 4.2 | 4.2 | 4.2 |
| 2. Frozen blood sample-E | Immediately after dilution | 4.3 | 4.4 | 4.4 | 4.3 | 3.8 |
| | 60 minutes after dilution | 4.4 | 4.4 | 4.5 | 4.4 | 4.7 |

(Example 7)

A hemoglobin sample was prepared by the following method, and HbA1c % was determined. Table 4 shows the results.

(1) Production of hemoglobin preparation solution

In purified water, 0.4 wt% of Tween 20 serving as a surfactant, 0.08 wt% of sodium azide serving as a preservative, 0.08 wt% of tetrasodium EDTA tetrahydrate serving as a preservative, and 0.08 wt% of dipotassium EDTA dihydrate serving as a preservative were dissolved to produce a hemoglobin preparation solution having a pH of 7.5.

(2) Preparation of hemoglobin sample

[I] Lyophilized hemoglobin-M

To lyophilized hemoglobin-M, 500 $\mu$L of purified water was added and the resulting solution was left to stand at room temperature for 30 minutes to obtain a 40 g/L high-concentration hemoglobin solution. Twenty microliters of the high-concentration hemoglobin solution was diluted with 1000 $\mu$L of the hemoglobin preparation solution to obtain an about 0.8 g/L hemoglobin sample.

[II] Lyophilized hemoglobin-N

To lyophilized hemoglobin-N, 500 $\mu$L of purified water was added and the resulting solution was left to stand at room temperature for 30 minutes to obtain a 40 g/L high-concentration hemoglobin solution. Twenty microliters of the high-concentration hemoglobin solution was diluted with 1000 $\mu$L of the hemoglobin preparation solution to obtain an about 0.8 g/L hemoglobin sample.

[III] Frozen blood sample-K

A blood sample stored at -80°C for two months or more was left to stand at room temperature for 20 minutes for thawing. The blood sample was diluted with the hemoglobin preparation solution to obtain an about 1 g/L hemoglobin sample.

[IV] Frozen blood sample-L

A blood sample stored at -80°C for two months or more was left to stand at room temperature for 20 minutes for thawing. The blood sample was diluted with the hemoglobin preparation solution to obtain an about 1 g/L hemoglobin sample.

(3) Measurement of HbA1c %

The hemoglobin sample was injected into a liquid chromatograph to which a cation exchange column was connected immediately after the dilution with the hemoglobin preparation solution, that is, within five minutes. Hemoglobin components were separated using succinate buffers having pH of 5.4 and detected at 415 nm. HbA1c % was calculated from the calculation formula A.

(Example 8)

A hemoglobin sample was prepared by the following method, and HbA1c % was determined. Table 4 shows the results.

(1) Production of hemoglobin preparation solution

The same process as in Example 7 was performed, except that the concentration of Tween 20 was changed to 0.2 wt%.
The preparation of a hemoglobin sample (2) and the measurement of HbA1c % (3) were performed in the same manner as in Example 7.

(Example 9; useful for understanding the invention)

A hemoglobin sample was prepared by the following method, and HbA1c % was determined. Table 4 shows the results.

(1) Production of hemoglobin preparation solution

The same process as in Example 7 was performed, except that the concentration of Tween 20 was changed to 0.05 wt%.
The preparation of a hemoglobin sample (2) and the measurement of HbA1c % (3) were performed in the same manner as in Example 7.

(Comparative Example 7)

A hemoglobin sample was prepared by the following method, and HbA1c % was determined. Table 4 shows the results.

(1) Production of hemoglobin preparation solution

The same process as in Example 7 was performed, except that the concentration of Tween 20 was changed to 0.5 wt%.
The preparation of a hemoglobin sample (2) and the measurement of HbA1c % (3) were performed in the same manner as in Example 7.

(Comparative Example 8)

A hemoglobin sample was prepared by the following method, and HbA1c % was determined. Table 5 shows the results.

(1) Production of hemoglobin preparation solution

In purified water, 0.08 wt% of sodium azide serving as a preservative, 0.08 wt% of tetrasodium EDTA tetrahydrate serving as a preservative, and 0.08 wt% of dipotassium EDTA dihydrate serving as a preservative were dissolved to produce a hemoglobin preparation solution having a pH of 7.5.
The preparation of a hemoglobin sample (2) and the measurement of HbA1c % (3) were performed in the same manner as in Example 7.

(Comparative Example 9)

A hemoglobin sample was prepared by the following method, and HbA1c % was determined. Table 4 shows the results.

(1) Production of hemoglobin preparation solution

In purified water, 0.5 wt% of Triton X-100 serving as a surfactant, 0.08 wt% of sodium azide serving as a preservative, 0.08 wt% of tetrasodium EDTA tetrahydrate serving as a preservative, and 0.08 wt% of dipotassium EDTA dihydrate serving as a preservative were dissolved to produce a hemoglobin preparation solution having a pH of 7.5.
The preparation of a hemoglobin sample (2) and the measurement of HbA1c % (3) were performed in the same manner as in Example 7. Herein, HbA1c % of the lyophilized hemoglobin-M in which Px was detected was calculated from the calculation formula B.

(Comparative Example 10)

A hemoglobin sample was prepared by the following method, and HbA1c % was determined. Table 4 shows the results.

(1) Production of hemoglobin preparation solution

The same process as in Comparative Example 9 was performed, except that the concentration of Triton X-100 was changed to 0.4 wt%.
The preparation of a hemoglobin sample (2) and the measurement of HbA1c % (3) were performed in the same manner as in Comparative Example 9.

(Comparative Example 11)

A hemoglobin sample was prepared by the following method, and HbA1c % was determined. Table 4 shows the results.

(1) Production of hemoglobin preparation solution

The same process as in Comparative Example 9 was performed, except that the concentration of Triton X-100 was changed to 0.2 wt%.
The preparation of a hemoglobin sample (2) and the measurement of HbA1c % (3) were performed in the same manner as in Comparative Example 9.

(Comparative Example 12)

A hemoglobin sample was prepared by the following method, and HbA1c % was determined. Table 4 shows the results.

(1) Production of hemoglobin preparation solution

The same process as in Comparative Example 9 was performed, except that the concentration of Triton X-100 was changed to 0.05 wt%.
The preparation of a hemoglobin sample (2) and the measurement of HbA1c % (3) were performed in the same manner as in Example 7.
As shown in Comparative Example 7, the case where the concentration of an ester ether-type nonionic surfactant that absorbs light having a wavelength of 415 nm exceeds 0.5 wt% is not preferred because the influence on HbA1c % is small, but a peak derived from the surfactant is detected in front of the peak of HbA1a. Furthermore, as is clear from Comparative Example 9 to Comparative Example 12, the ether-type surfactant affects HbA1c % even at low concentration.

(Example 10)

A hemoglobin sample was prepared by the following method, and the change in HbA1c % over time after dilution was observed. Table 5 shows the results.

(1) Hemoglobin preparation solution

In purified water, 0.1 wt% of Tween 20 serving as a surfactant, 0.075 wt% of sodium azide serving as a preservative, 0.075 wt% of tetrasodium EDTA tetrahydrate serving as a preservative, and 0.075 wt% of dipotassium EDTA dihydrate serving as a preservative were dissolved to produce a hemoglobin preparation solution. The hemoglobin preparation solution had a pH of 7.6.

(2) Preparation of hemoglobin sample

[I] Blood sample-D

A blood sample-D taken within 24 hours was diluted with the hemoglobin preparation solution to obtain an about 1 g/L hemoglobin sample.

[II] Frozen blood sample-E

A blood sample-E stored at -80°C for two months or more was left to stand at room temperature for 20 minutes for thawing. The blood sample was diluted with the hemoglobin preparation solution to obtain an about 1 g/L hemoglobin sample.

[III] Lyophilized hemoglobin-F

To lyophilized hemoglobin-F, 1000 $\mu$L of the hemoglobin preparation solution was added, and suction and discharge were repeatedly performed three times using a micropipette to obtain an about 0.8 g/L hemoglobin sample.

[IV] Lyophilized hemoglobin-G

To lyophilized hemoglobin-G, 500 $\mu$L of purified water was added and the resulting solution was left to stand at room temperature for 30 minutes to obtain a 40 g/L high-concentration hemoglobin solution. Twenty microliters of the high-concentration hemoglobin solution was diluted with 1000 $\mu$L of the hemoglobin preparation solution to obtain an about 0.8 g/L hemoglobin sample. The lyophilized hemoglobin-G was obtained from the same manufacturer as that of the lyophilized hemoglobin-M used in Example 7.

[V] Frozen hemoglobin-H

A frozen hemoglobin-H stored at -80°C was left to stand at room temperature for 10 minutes for thawing. The frozen hemoglobin-H was obtained from the same manufacturer as that of the frozen hemoglobin solution-J used in Example 3. Six microliters of the thawed liquid was diluted with 1000 $\mu$L of the hemoglobin preparation solution to obtain an about 0.8 g/L hemoglobin sample.

(3) Measurement of HbA1c %

The hemoglobin sample was injected into a liquid chromatograph to which a cation exchange column was connected immediately after the dissolution or dilution with the hemoglobin preparation solution, that is, within five minutes. Hemoglobin components were separated using succinate buffers having pH of 5.4 and detected at 415 nm. Since Px was not observed in the obtained chromatogram, HbA1c % was calculated from the calculation formula A as in Example 4.

[Table 4]

| | Comparative Example 7 | Example 7 | Example 8 | Example 9 | Comparative Example 8 |
|---|---|---|---|---|---|
| Tween 20 concentration (wt%) | 0.5 | 0.4 | 0.2 | 0.05 | - |
| HbA1c % | 1. Lyophilized hemoglobin-M | 4.2 | 4.2 | 4.2 | 4.2 | 4.2 |
| | 2. Lyophilized hemoglobin-N | 8.7 | 8.7 | 8.6 | 8.6 | 8.7 |
| | 3. Blood sample-K | 4.8 | 4.8 | 4.8 | 4.7 | 4.7 |
| | 4. Blood sample-L | 7.9 | 7.9 | 7.9 | 7.9 | 7.8 |

| | Comparative Example 9 | Comparative Example 10 | Comparative Example 11 | Comparative Example 12 | Comparative Example 8 |
|---|---|---|---|---|---|
| Triton X-100 concentration (wt%) | 0.5 | 0.4 | 0.2 | 0.05 | - |
| HbA1c % | 1. Lyophilized hemoglobin-M | <u>3.9</u> | <u>3.9</u> | <u>3.9</u> | 4.4 | 4.2 |
| | 2. Lyophilized hemoglobin-N | 9.1 | 9.1 | 9.0 | 8.9 | 8.7 |
| | 3. Frozen blood sample-K | 4.9 | 4.9 | 5.0 | 4.9 | 4.7 |
| | 4. Frozen blood sample-L | 8.0 | 8.1 | 8.1 | 8.0 | 7.8 |

[Table 5]

| | Elapsed time after dissolution or dilution with hemoglobin preparation | |
|---|---|---|
| | Immediate | 60 minutes |
| 1. Blood sample-D | 4.1 | 4.1 |
| 2. Frozen blood sample-E | 4.4 | 4.4 |
| 3. Lyophilized hemoglobin-F | 5.0 | 5.0 |
| 4. Lyophilized hemoglobin-G | 4.2 | 4.2 |
| 5. Frozen hemoglobin-H | 5.1 | 5.1 |

In general, a commercially available calibrator is used by a method in which a hemoglobin preparation solution for dissolving and diluting lyophilized hemoglobin is added to a calibrator, the resulting solution is left to stand for a predetermined time, 30 minutes in many cases, and then measurement is performed. In Example 10, however, it has been confirmed that a hemoglobin-containing sample can be injected into the column within five minutes after the hemoglobin preparation solution is added to the lyophilized hemoglobin-F. In other words, it has been confirmed that immediately after a measurer adds the hemoglobin preparation solution according to the present invention to the lyophilized hemoglobin, the measurer can introduce the sample into an instrument and can start the calibration of the instrument. This shows that a hemoglobin measuring instrument equipped with a container containing lyophilized hemoglobin can automatically add the hemoglobin preparation solution according to the present invention to the container containing a hemoglobin-containing sample, perform suspension and suction, and immediately inject the sample into a column for calibration without human operation.

## Claims

1. Use of a hemoglobin preparation solution

for preparing a calibrator, a control, and a blood sample for measuring hemoglobins from a hemoglobin-containing sample in a liquid chromatography method, or
for dissolving and diluting lyophilized hemoglobin for measuring hemoglobins from a hemoglobin-containing sample in a liquid chromatography method,
wherein the hemoglobin preparation solution comprises 0.05 to 0.4 wt% of at least one ester ether-type nonionic surfactant after the hemoglobin-containing sample is prepared, and wherein the hemoglobin preparation solution does not contain an ether-type nonionic surfactant.

2. The use of a hemoglobin preparation solution according to Claim 1, wherein the ester ether-type nonionic surfactant is at least one of polyoxyethylene (20) sorbitan monolaurate and polyoxyethylene (20) sorbitan monooleate.

3. The use of a hemoglobin preparation solution according to Claim 1 or 2, comprising at least one preservative.

4. The use of the hemoglobin preparation solution according to Claim 3, wherein the preservative is at least one of ethylenediaminetetraacetic acid, glycol ether diaminetetraacetic acid, ethylenediamine-N,N'-disuccinic acid, sodium salts of ethylenediaminetetraacetic acid, potassium salts of ethylenediaminetetraacetic acid, sodium salts of ethylenediamine-N,N'-disuccinic acid, and sodium azide.

5. A liquid chromatography method for measuring hemoglobin, comprising measuring hemoglobins in a hemoglobin-containing sample using a calibrator, a control, and a blood sample prepared by using the hemoglobin preparation solution defined in any one of Claims 1 to 4.

6. A liquid chromatography method for measuring hemoglobins from a hemoglobin-containing sample, the method comprising dissolving and diluting lyophilized hemoglobin with the preparation solution defined in any one of Claims 1 to 4 and then immediately performing introduction into a column.

## Patentansprüche

1. Verwendung einer Hämoglobinzubereitungslösung

zur Herstellung eines Kalibrators, einer Kontrolle und einer Blutprobe zur Messung von Hämoglobinen aus einer hämoglobinhaltigen Probe in einem Flüssigchromatographieverfahren, oder
zum Lösen und Verdünnen von lyophilisiertem Hämoglobin zur Messung von Hämoglobinen aus einer hämoglobinhaltigen Probe in einem Flüssigchromatographieverfahren,
wobei die Hämoglobinzubereitungslösung 0,05 bis 0,4 Gew.-% mindestens eines nichtionischen Tensids vom Ether-Typ umfasst, nachdem die Hämoglobin-enthaltende Probe hergestellt wurde, und wobei die Hämoglobinzubereitungslösung kein nichtionisches Tensid vom Ether-Typ enthält.

2. Verwendung einer Hämoglobinzubereitungslösung nach Anspruch 1, wobei das nichtionische Tensid vom Esterether-Typ mindestens eines von Polyoxyethylen(20)sorbitanmonolaurat und Polyoxyethylen(20)sorbitanmonooleat ist.

3. Verwendung einer Hämoglobinzubereitungslösung nach Anspruch 1 oder 2, die mindestens ein Konservierungsmittel umfasst.

4. Verwendung der Hämoglobinzubereitungslösung nach Anspruch 3, wobei das Konservierungsmittel mindestens eines der folgenden ist: Ethylendiamintetraessigsäure, Glykoletherdiamintetraessigsäure, Ethylendiamin-N,N'-bernsteinsäure, Natriumsalze der Ethylendiamintetraessigsäure, Kaliumsalze der Ethylendiamintetraessigsäure, Natriumsalze der Ethylendiamin-N,N'-bernsteinsäure und Natriumazid.

5. Flüssigchromatographieverfahren zur Messung von Hämoglobin, umfassend die Messung von Hämoglobinen in einer hämoglobinhaltigen Probe unter Verwendung eines Kalibrators, einer Kontrolle und einer Blutprobe, die unter Verwendung der in einem der Ansprüche 1 bis 4 definierten Hämoglobinzubereitungslösung hergestellt wurden.

**6.** Flüssigchromatographieverfahren zur Messung von Hämoglobinen aus einer hämoglobinhaltigen Probe, wobei das Verfahren das Auflösen und Verdünnen von lyophilisiertem Hämoglobin mit der in einem der Ansprüche 1 bis 4 definierten Zubereitungslösung und die anschließende sofortige Einführung in eine Säule umfasst.

**Revendications**

**1.** Utilisation d'une solution d'une préparation d'hémoglobine

pour préparer un calibrateur, un témoin et un échantillon sanguin pour mesurer les hémoglobines à partir d'un échantillon contenant de l'hémoglobine dans une méthode de chromatographie en phase liquide, ou
pour dissoudre et diluer une hémoglobine lyophilisée pour mesurer les hémoglobines provenant d'un échantillon contenant de l'hémoglobine dans une méthode de chromatographie en phase liquide,
la solution d'une préparation d'hémoglobine comprenant 0,05 à 0,4 % en poids d'au moins un tensioactif non ionique de type ester-éther après préparation de l'échantillon contenant de l'hémoglobine, et la solution d'une préparation d'hémoglobine ne contenant pas de tensioactif non ionique de type éther.

**2.** Utilisation d'une solution d'une préparation d'hémoglobine selon la revendication 1, dans laquelle le tensioactif non ionique de type ester-éther est au moins l'un du monolaurate de sorbitan polyoxyéthyléné (20) et du monooléate de sorbitan polyoxyéthyléné (20).

**3.** Utilisation d'une solution d'une préparation d'hémoglobine selon la revendication 1 ou 2, comprenant au moins un conservateur.

**4.** Utilisation d'une solution d'une préparation d'hémoglobine selon la revendication 3, le conservateur étant au moins l'un parmi l'acide éthylènediaminetétraacétique, l'acide glycoléther-diaminetétraacétique, l'acide éthylènediamine-N,N'-disuccinique, les sels de sodium de l'acide éthylènediaminetétraacétique, les sels de potassium de l'acide éthylènediaminetétraacétique, les sels de sodium de l'acide éthylènediamine-N,N'-disuccinique et l'azoture de sodium.

**5.** Méthode de chromatographie en phase liquide pour mesurer l'hémoglobine, comprenant la mesure des hémoglobines dans un échantillon contenant de l'hémoglobine par utilisation d'un calibrateur, d'un témoin et d'un échantillon sanguin préparé par utilisation de la solution d'une préparation d'hémoglobine définie dans l'une quelconque des revendications 1 à 4.

**6.** Méthode de chromatographie en phase liquide pour mesurer les hémoglobines à partir d'un échantillon contenant de l'hémoglobine, la méthode comprenant la dissolution et la dilution de l'hémoglobine lyophilisée avec la solution d'une préparation définie dans l'une quelconque des revendications 1 à 4, puis la mise en oeuvre immédiate d'une introduction dans une colonne.

Fig. 1

(A) IMMEDIATELY AFTER DILUTION

(B) 30 MINUTES AFTER DILUTION

HbA0

HbA1c

L-A1c

HbF

HbA1b

HbA1a

0    0.5

TIME (MIN)

0    0.5

TIME (MIN)

EP 3 483 603 B1

Fig. 2

(A) IMMEDIATELY AFTER DILUTION        (B) 30 MINUTES AFTER DILUTION

TIME (MIN)        TIME (MIN)

Fig. 3

Fig. 4

(A) EXAMPLE 9

HbA1c

HbA1a

TIME (MIN)

(B) COMPARATIVE EXAMPLE 8 HbA1c

TIME (MIN)

(C) COMPARATIVE EXAMPLE 7

HbA1c

Tween20

HbA1a

TIME (MIN)

(D) COMPARATIVE EXAMPLE 12

HbA1c

TIME (MIN)

EP 3 483 603 B1

EP 3 483 603 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4061365 B
- JP 6331629 A
- JP 2009133654 A
- EP 2360471 A1

**Non-patent literature cited in the description**

- EFFECTS OF TWEEN 80 AND SUCROSE ON ACUTE SHORT-TERM STABILITY AND LONG-TERM STORAGE AT - 20°C OF A RECOMBINANT HEMOGLOBIN. **KERWIN B A et al.** JOURNAL OF PHARMACEUTICAL SCIENCES. AMERICAN PHARMACEUTICAL ASSOCIATION, 01 September 1998, vol. 87, 1062-1068

- **C G DUCK-CHONG.** Differential effect of detergents on the alkaline denaturation of haemoglobin in maternal and fetal blood, with particular reference to Triton X-100. *JOURNAL OF CLINICAL PATHOLOGY, GB,* 01 August 1983, vol. 36 (8), 910-914